(19) **Europäisches Patentamt / European Patent Office / Office européen des brevets**

(11) **EP 2 776 663 B1**

(12) # EUROPEAN PATENT SPECIFICATION

(45) Date of publication and mention of the grant of the patent:
**22.07.2020 Bulletin 2020/30**

(51) Int Cl.:
*G01N 1/00* *(2006.01)*  *G01N 33/24* *(2006.01)*
*G01V 9/00* *(2006.01)*  *E21B 43/16* *(2006.01)*

(21) Application number: **12847565.4**

(22) Date of filing: **27.08.2012**

(86) International application number:
**PCT/US2012/052542**

(87) International publication number:
**WO 2013/070304 (16.05.2013 Gazette 2013/20)**

(54) **METHOD FOR DETERMINING THE PRESENCE AND LOCATION OF A SUBSURFACE HYDROCARBON ACCUMULATION AND THE ORIGIN OF THE ASSOCIATED HYDROCARBONS**

VERFAHREN ZUR BESTIMMUNG DER PRÄSENZ UND DES ORTES EINER UNTERIRDISCHEN KOHLENWASSERSTOFFANSAMMLUNG UND DER HERKUNFT DIESER KOHLENWASSERSTOFFE

PROCÉDÉ POUR DÉTERMINER LA PRÉSENCE ET L'EMPLACEMENT D'UNE ACCUMULATION D'HYDROCARBURES SOUS LA SURFACE ET L'ORIGINE DES HYDROCARBURES ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2011 US 201161558822 P**

(43) Date of publication of application:
**17.09.2014 Bulletin 2014/38**

(73) Proprietor: **ExxonMobil Upstream Research Company**
**Spring TX 77389 (US)**

(72) Inventors:
• **POTTORF, Robert J.**
**Houston, TX 77055 (US)**
• **LAWSON, Michael**
**Houston, TX 77019 (US)**
• **MAY, Steven R.**
**Bellaire, TX 77401 (US)**
• **DREYFUS, Sebastien**
**Houston, TX 77008 (US)**
• **RAMAN, Sumathy**
**Annandale, NJ 08801 (US)**

(74) Representative: **ExxonMobil Chemical Europe Inc.**
**IP Law Europe**
**Hermeslaan 2**
**1831 Machelen (BE)**

(56) References cited:
**WO-A2-2007/008932** **US-A1- 2010 279 290**
**US-A1- 2011 088 895** **US-B1- 7 704 746**

• **1 HOHL: "Energy, Environment and Climate Workshop", SHELL'S BELLAIRE TECHNOLOGY CENTER (BTC), 22 July 2010 (2010-07-22), pages 1-38, XP055152533,**
• **EILER ET AL: "''Clumped-isotope'' geochemistry-The study of naturally-occurring, multiply-substituted isotopologues", EARTH AND PLANETARY SCIENCE LETTERS, NORTH HOLLAND PUBL., CO, NL, vol. 262, no. 3-4, 12 October 2007 (2007-10-12), pages 309-327, XP022532839, ISSN: 0012-821X, DOI: 10.1016/J.EPSL.2007.08.020**
• **K. W. HUNTINGTON ET AL: "Use of Clumped-Isotope Thermometry To Constrain the Crystallization Temperature of Diagenetic Calcite", JOURNAL OF SEDIMENTARY RESEARCH, vol. 81, no. 9, 19 August 2011 (2011-08-19), pages 656-669, XP055201016, ISSN: 1527-1404, DOI: 10.2110/jsr.2011.51**
• **HOHL ET AL.: 'DCO Energy, Environment and Climate Workshop' SHELL'S BELLAIRE TECHNOLOGY CENTER (BTC) 22 July 2010, HOUSTON, TX, pages 1 - 38, XP055152533 Retrieved from the Internet: <URL:https:l/dco.gl.ciw.edu/sites/dco.gl.ciw.edu/files/images/DCO%20EEC%20White%20Paper_Final,pdf> [retrieved on 2012-10-22]**

**(Cont. next page)**

• **OZGUL ET AL.: 'Geochemical Assessment of Gaseous Hydrocarbons: Mixing of Bacterial and Thermogenic Methane in the Deep Subsurface Petroleum System, Gulf of Mexico Continental Slope (Thesis)', [Online] August 2002, pages 1 - 167, XP055067583 Retrieved from the Internet: <URL:http://repository.tamu.edu/bitstream/handle/1969.1/223/etd-07182002-124338-l.pdf?sequence=1> [retrieved on 2012-10-22]**

**Description**

**FIELD OF THE INVENTION**

Embodiments of the present disclosure relate generally to the field of geochemistry. More particularly, the present disclosure relates to systems and methods for determining the origin and storage temperature (and hence depth) of subsurface hydrocarbon accumulations.

**BACKGROUND**

This section is intended to introduce various aspects of the art, which may be associated with exemplary embodiments of the present disclosure. This discussion is believed to assist in providing a framework to facilitate a better understanding of particular aspects of the present invention. Accordingly, it should be understood that this section should be read in this light, and not necessarily as admissions of prior art.

The major components required for the presence of subsurface hydrocarbon accumulations in a sedimentary basin are (1) the generation and expulsion of liquid hydrocarbons from a source rock, (2) migration of liquid hydrocarbons to and accumulation in a reservoir, (3) a trap and a seal to prevent significant leakage of hydrocarbons from the reservoir.

At present, reflection seismic is the dominant technology for the identification of hydrocarbon accumulations. This technique has proved successful in identifying structures that may host hydrocarbon accumulations, and in some cases have been used to image the hydrocarbon fluids within subsurface accumulations. However, in some cases this technology lacks the required fidelity to provide accurate assessments of the location of subsurface hydrocarbon accumulations due to poor imaging of the subsurface. Additionally, it is not easy to differentiate the presence and types of hydrocarbons from other fluids in the subsurface by remote measurements.

Current non-seismic hydrocarbon detection technologies do not significantly improve our ability to identify the location of a hydrocarbon accumulation. For example, seepage of hydrocarbons at the sea floor or on land provides some indication of an active or working hydrocarbon system where hydrocarbons have been generated and expulsed during the thermal maturation of a source rock at depth, and have migrated via more or less complex migration pathways to the surface. However, it is difficult from current non-seismic technologies to determine whether such hydrocarbon seepages migrated directly from a source rock or from a hydrocarbon accumulation, and it is not possible to locate subsurface accumulations associated with seeps.

As such, there is a need for additional techniques that can more effectively detect the presence and the location of hydrocarbon accumulations in the subsurface. In particular, a relatively inexpensive and rapid method for determining the presence and location of a subsurface hydrocarbon accumulation and the origin of the associated hydrocarbons (i.e. source facies and thermal maturity of the source rock that have generated these hydrocarbons) would provide a valuable tool that could be used in hydrocarbon exploration at all business stage maturity levels, from frontier exploration to extension of proven plays or high-grading prospects in proven plays.

Hohl, D., and Mailhiot, C., DCO Energy, Environment and Climate Workshop, 22 July 2010, pages 1-38, describes the potential use of noble gas and isotopologue measurements in determining abiogenic vs. biogenic origins of hydrocarbons, and discloses that experimental confirmation or calibration of theoretical predictions may be a key in the use of isotopic distribution techniques.

D2 Eiler, J.M., "Clumped-isotope" geochemistry - The study of naturally occurring multiply substituted isotopologues, Earth and Planetary Science Letters (2007) pages 309-327, describes that, in the field of geochemistry, deviations from stochastic distributions of isotopes result from enhanced thermodynamic stability of heavy isotope 'clumps', slower kinetics of reactions requiring the breakage of bonds between heavy isotopes, the mass dependence of diffusive and thermos-gravitational fractionations, mixing between components that differ from one another in bulk isotopic composition, biochemical and petrochemical fractionations that may reflect combinations of these simpler mechanisms, and, in some cases, other process we do not yet understand. This document describes potential applications of clumped isotope geochemistry, such as analysis of ancient carbonates and reconstruction of marine temperatures and terrestrial ground temperatures. This document also describes that this field could be extended to encompass volatile hydrocarbons.

WO2007008932 A2 relates to methods of determining natural gas paleotemperature, origin, and maturity by measuring $^{13}CDH3$ methane double isotopologues. and optical methods to determine both $^{13}CDH_3$ and $^{13}C^{18}O^{16}O$ double isotopologues. In a method to determine carbon and hydrogen isotopes in methane, ethane and propane by using a gas chromatography followed by a combustion and optical methods is disclosed.

Huntington, et al. Use of clumped-isotope thermometry to constrain the crystallization temperature of diagenetic calcite. Journal of Sedimentary Research. 2011. 656-669, describes an approach to estimating the crystallization temperature of diagenetic calcites using clumped-isotope thermometry, a paleothermometer based on the $^{13}C-^{18}O$-bond enrichment in carbonates. The combination of independent constraints on thermal history with clumped isotope thermometry, petrographic observations and oxygen isotopic data provides a basis for estimation of the temperature and timing of diagentic events and fluid sources. Addition of clumped-isotopic thermometry is used to demonstrate the temperatures of phase transformations. The results illustrate the potential for clumped isotope thermometry to constrain conditions of diagenesis and guide interpretations that would not be possible on the basis of conventional stable-isotopic and petrographic data alone.

SUMMARY

According to disclosed aspects and methodologies, a system and method are provided according to independent claims 1, 10. Preferred embodiments are provided in the dependent claims.

Features and advantages of the present disclosure will be readily apparent upon consideration of the following description in conjunction with the accompanying drawings.

**BRIEF DESCRIPTION OF THE DRAWINGS**

Figure 1 is a side elevational view of a seafloor;
Figure 2 is a flow diagram of a method in accordance with disclosed methodologies and techniques;
Figure 3 is a graph of isotopologue concentration versus temperature;
Figure 4 is a block diagram of a computer system according to disclosed methodologies and techniques; and
Figure 5 is a flow diagram representing machine-readable instructions according to disclosed methodologies and techniques.

**DETAILED DESCRIPTION**

Various terms as used herein are defined below. To the extent a term used in a claim is not defined below, it should be given the definition persons in the pertinent art have given that term in the context in which it is used.

As used herein, "a" or "an" entity refers to one or more of that entity. As such, the terms "a" (or "an"), "one or more", and "at least one" can be used interchangeably herein unless a limit is specifically stated.

As used herein, the terms "comprising," "comprises," "comprise," "comprised," "containing," "contains," "contain," "having," "has," "have," "including," "includes," and "include" are open-ended transition terms used to transition from a subject recited before the term to one or more elements recited after the term, where the element or elements listed after the transition term are not necessarily the only elements that make up the subject.

As used herein, "exemplary" means exclusively "serving as an example, instance, or illustration." Any embodiment described herein as exemplary is not to be construed as preferred or advantageous over other embodiments.

As used herein "hydrocarbons" are generally defined as molecules formed primarily of carbon and hydrogen atoms such as oil and natural gas. Hydrocarbons may also include other elements or compounds, such as, but not limited to, halogens, metallic elements, nitrogen, oxygen, sulfur, hydrogen sulfide ($H_2S$) and carbon dioxide ($CO_2$). Hydrocarbons may be produced from hydrocarbon reservoirs through wells penetrating a hydrocarbon containing formation. Hydrocarbons derived from a hydrocarbon reservoir may include, but are not limited to, petroleum, kerogen, bitumen, pyrobitumen, asphaltenes, tars, oils, natural gas, or combinations thereof. Hydrocarbons may be located within or adjacent to mineral matrices within the earth, termed reservoirs. Matrices may include, but are not limited to, sedimentary rock, sands, silicilytes, carbonates, diatomites, and other porous media.

As used herein, "hydrocarbon production" refers to any activity associated with extracting hydrocarbons from a well or other opening. Hydrocarbon production normally refers to any activity conducted in or on the well after the well is completed. Accordingly, hydrocarbon production or extraction includes not only primary hydrocarbon extraction but also secondary and tertiary production techniques, such as injection of gas or liquid for increasing drive pressure, mobilizing the hydrocarbon or treating by, for example chemicals or hydraulic fracturing the wellbore to promote increased flow, well servicing, well logging, and other well and wellbore treatments.

As used herein the term "isotope" refers to one of two or more atoms with the same atomic number but with different numbers of neutrons. Hydrocarbon molecules may contain a variety of isotopes. Hydrocarbon molecules contain both carbon and hydrogen atoms. Carbon can be present in the molecule as one of two stable isotopes: $^{12}C$, which has 6 protons and 6 neutrons (shown herein as C); and, in much lower concentrations, $^{13}C$, which has 6 protons and 7 neutrons. Similarly, hydrogen can be present in a molecule as one of two stable isotopes: H, which contains 1 proton but no neutron; and, in much lower concentrations, Deuterium (D), which has 1 proton and 1 neutron.

As used herein the term "signatures" refers to the relative abundances, concentrations and/or ratios of various elements, isotopes and isotopologues of a given species.

As used herein the term "isotopologue" refers generally to molecules that have the same chemical composition, but have a different isotopic signature; for example, methane contains 1 atom of carbon and four atoms of hydrogen. Each atom in the methane structure can contain one of the two stable isotopes of that atom, and as such there are 10 possible isotopologues of methane.

As used herein the term "multiply substituted isotopologue" refers generally to an isotopologue that contains at least two rare isotopes in its structure; for example, a multiply substituted methane isotopologue must contain one $^{13}C$ atom and one D atom, or at least 2 D atoms in the absence of a $^{13}C$ atom.

As used herein the term "clumped isotopologue" refers generally to an isotopologue that contains at least two rare isotopes that share a common chemical bond in its structure; for example, a clumped isotopologue of methane must one $^{13}C$ atom that shares a chemical bond with at least one D atom.

As used herein the term "stochastic distribution" refers generally to a system where the stable isotopes in a given population of molecules are distributed randomly among all possible isotopologues of a given species. This stochastic distribution is the reference frame from which deviations are measured and is used to provide a baseline to identify anomalies that may be associated with secondary isotope exchange processes.

While for purposes of simplicity of explanation, the illustrated methodologies are shown and described as a series of blocks, it is to be appreciated that the methodologies are not limited by the order of the blocks, as some blocks can occur in different orders and/or concurrently with other blocks from that shown and described. Moreover, less than all the illustrated blocks may be required to implement an example methodology. Blocks may be combined or separated into multiple components. Furthermore, additional and/or alternative methodologies can employ additional, not illustrated blocks. While the figures illustrate various serially occurring actions, it is to be appreciated that various actions could occur concurrently, substantially in parallel, and/or at substantially different points in time.

In the following section, specific embodiments of the present invention are described in connection with disclosed aspects and techniques. However, to the extent that the following description is specific to a particular aspect, technique, or a particular use, this is intended to be for exemplary purposes only. Accordingly, the invention is not limited to the disclosed aspects and techniques described below, but rather includes all alternatives and modifications falling within the scope of the appended claims.

According to aspects of the disclosed methodologies and techniques, the clumped isotope signature of numerous co-existing isotopologues of hydrocarbons can be used to determine (i) the presence and depth of a subsurface hydrocarbon accumulation, and (ii) through integration with more conventional isotopic and molecular geochemistry techniques, the origin of associated hydrocarbons. With further integration with conventional geophysical techniques such as seismic reflection, the precise location (the depth plus lateral location) of the subsurface hydrocarbon accumulation can be identified.

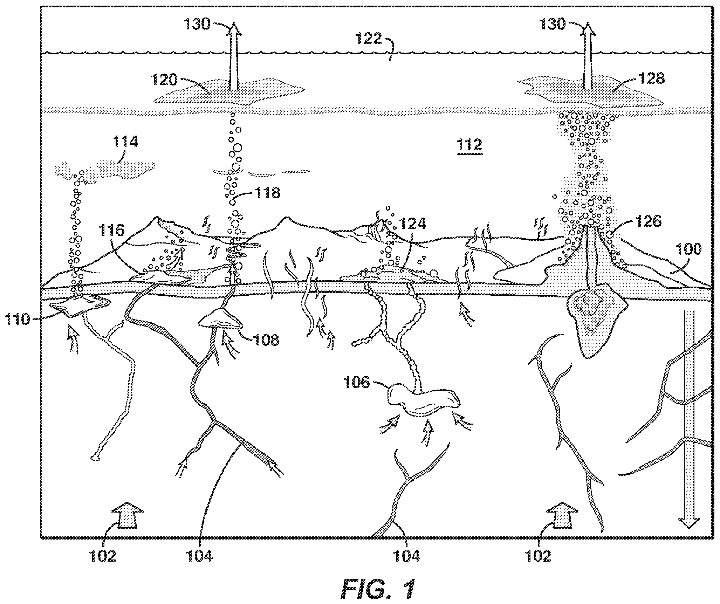

Figure 1 is a diagram illustrating the numerous subsurface sources and migration pathways of hydrocarbons present at or escaping from seeps on the ocean floor 100. Hydrocarbons 102 generated at source rock (not shown) migrate upward through faults and fractures 104. If limited by subsurface geology the hydrocarbons may be trapped in hydrocarbon accumulations such as a gas reservoir 106, an oil/gas reservoir 108, or a gas hydrate accumulation 110. Hydrocarbons seeping from the gas hydrate accumulation may dissolve into methane in the ocean 112 as shown at 114, or may remain as a gas hydrate on the ocean floor 100 as shown at 116. Alternatively, oil or gas from oil/gas reservoir 108 may seep into the ocean, as shown at 118, and form an oil slick 120 on the ocean surface 122. Gas leaking from gas reservoir 106 may form a bacterial mat 124 that may generate biogenic hydrocarbon gases, which is another sign of seepage. Still another method of hydrocarbon seepage is via a mud volcano 126, which can form an oil slick 128 on the ocean surface. Oil slicks 120 and 128 or methane gas 130 emitted therefrom are signs of hydrocarbon seepage that are, in turn, signs of possible subsurface hydrocarbon accumulation. The signatures measured from each of these seeps may be interrogated according to disclosed methodologies and techniques herein to discriminate between the different origins of hydrocarbons encountered at these seeps. In particular, this invention will discriminate between hydrocarbons that have migrated directly to the surface seep without encountering a structure/seal within which they can be stored (source 1) and hydrocarbons that have escaped from a subsurface accumulation (source 2). If the presence and location of such a hydrocarbon accumulation can be identified, it is possible the hydrocarbons from such an accumulation can be extracted.

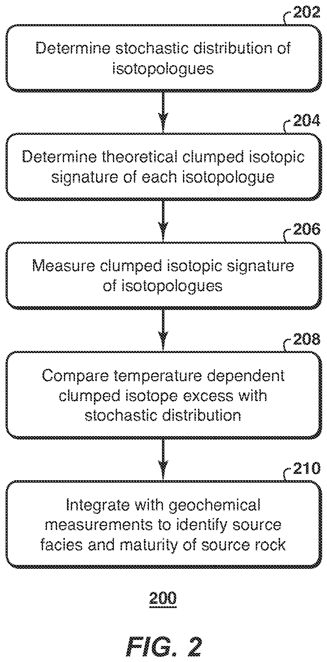

Figure 2 depicts a flow diagram of a method 200 for determining the (i) location of a subsurface hydrocarbon accumulation, and (ii) the source facies and thermal maturity of associated hydrocarbons sampled from a sea-floor seep. According to the method, at block 202 the stochastic distribution of isotopologues of a hydrocarbon species of interest is determined for a given bulk isotopic signature for that species. Determining the stochastic distribution of isotopologues requires knowledge of the bulk isotope signature of the species from which it derives. For example, if determining the stochastic distribution of isotopologues for methane, calculating the stochastic distribution requires the $^{13}C$ and D signatures of methane. The isotopic signature of hydrocarbon gases that are stored in a subsurface accumulation or that are present at seeps may reflect the isotopic signature of the gas generated from the source rock. As such, this signature may be concomitantly determined during the characterization of the hydrocarbons present at a seep and substituted directly in to the calculation of the stochastic distribution. There may be occasions, however, when the isotopic signature of gases may be altered due to various processes such as mixing with biogenic gas. In such instances, correction schemes such as that proposed by Chung et al., "Origin of gaseous hydrocarbons in subsurface environment: theoretical considerations of carbon isotope distribution", Chemical Geology, v. 71, p. 97-104 (1988), can be used to deconvolve such contributions and reach the initial primary isotope signature that should be used in the calculation of the stochastic distribution.

At block 204 ab-initio calculations are made to determine the theoretical clumped isotopic signature of each isotopologue of the hydrocarbon of interest. The ab-initio calculations conducted in the molecular modeling focus on a method for calculating the abundances of all isotopologues for any given hydrocarbon in a thermally equilibrated population of isotopologues. This method incorporates three linked algorithms. The first of these algorithms is able to select a subset of the isotopologues of any given species that can uniquely define the bulk isotopic composition of a given population of molecules (for example H/D ratio, including contributions from all isotopologues). The second algorithm is used to define the set of isotopic exchange reactions between all isotopologues for which calculation of an equilibrium constant is required. Finally, the third algorithm is used to calculate the selected equilibrium constants from molecular properties such as molecular mass, rotational constants, vibrational frequencies, anharmonicity corrections and vibration-rotation coupling constants. The latter parameters are calculated using high-level first principle calculations discussed below (e.g. coupled cluster singles, doubles and triples excitation approach using a very large correlation-consistent basis set).

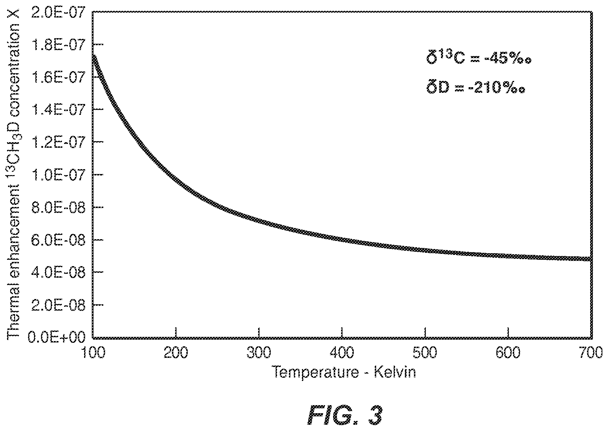

If methane, the primary chemical component of natural gases, is used as an example, it is possible to investigate the potential of forming the clumped doubly substituted isotopologue $^{13}CH_3D$, and the doubly substituted isotopologue $^{12}CH_2D_2$. As shown in Figure 3, in which the thermal enhancement of various concentrations of $^{13}CH_3D$ are plotted versus temperature, the modeled clumped isotope signatures of $^{13}CH_3D$ and $^{12}CH_2D_2$ vary with temperature. Indeed, it is possible to calculate the thermal dependence for any isotopologue of any hydrocarbon species given the isotopic signature.

If one considers the isotopologue $^{13}CH_3D$, its total relative abundance in bulk methane should be controlled by (a) temperature-independent randomly populated processes (stochastic distribution) and (b) thermal equilibrium isotopic exchange. The latter process is controlled or dependent on the surrounding temperature. These processes can be determined from first-principle quantum mechanical calculations (such as Couple-cluster Singles Doubles and Triples, CCSD(T) calculations or Density functional theory (DFT)) to investigate the $^{13}CH_3D$ formation, its thermodynamic equilibration and temperature dependence.

The concentration of the doubly-substituted methane isotopologues $N[^{13}CH_3D]_0$ relative to the total methane concentration $N[CH4]_0$ in a stochastic distribution can be calculated for any given relative concentrations of $^{13}C$ and D ($N[^{13}C]$ and $N[D]$)] from equation (1) below:

$$N[^{13}CH_3D]_0 = \frac{N[^{13}C]N[D]}{N[CH_4]_0} \qquad (1)$$

Any deviation between this modeled concentration and the measured concentration for a given isotopologue (discussed below) is merely a function of the temperature at which the species was stored assuming it reaches isotopic equilibrium for a given temperature over geologic timescales. The temperature dependent isotopic exchange of any species is governed by thermal equilibrium with a known equilibrium constant, $K_{eq}(T)$, and can be described for the examples above by the reaction:

$$^{13}CH_4 + CH_3D \leftrightarrow CH_4 + ^{13}CH_3D \ (K_{eq}) \qquad (2)$$

If the temperature dependent difference between a stochastic and non-stochastic distribution is given by $N[^{13}CH_3D]_T$, then following achieving thermal equilibrium, the concentration of the isotopologues involved in equation (2) can be described by the following equations.

$$N[^{13}CH_3D] = N[^{13}CH_3D]_0 + N[^{13}CH_3D]_T \qquad (3)$$

$$N[^{13}CH_4] = N[^{13}C] - N[^{13}CH_3D]_T N[^{13}CH_3D]_T \qquad (4)$$

$$N[CH_3D] = N[D] - N[^{13}CH_3D]_T \qquad (5)$$

$$N[CH_4] = N[CH_4]_0 - (N[^{13}CH_4]-N[^{13}CH_3D]_T) - (N[D]-N[^{13}CH_3D]_T) - N[D]-(N[^{13}CH_3D]_0 + N[^{13}CH_3D]_T) \qquad (6)$$

From equations (3)-(6) it is possible to describe an equilibrium constant for the initial reaction given in equation (2). The equilibrium constant can then be calculated for any given temperature from high-level quantum chemical calculations and using the Urey Model from partition function of products and reactants.

The total abundance of $N[^{13}CH_3D]$ can therefore be calculated from knowledge of $K_{eq}(T)$, $N[^{13}C]$ and $N[D]$, combining statistical and thermal equilibrium effects at any given temperature.

The example described above can be applied to determine the expected abundance of any isotopologue on which measurements can be made where the error associated with the measurement does not exceed the deviation from a purely stochastic distribution for a given temperature and primary bulk isotopic signature of the hydrocarbon species of interest.

Returning to Figure 2, at block 206 the clumped isotopic signature of the isotopologues of the hydrocarbons of interest is measured. The measurement of the absolute abundance of isotopologues for any given hydrocarbon requires knowledge of the molecular mass at which they are present, and hence requires knowledge of the actual identity of each possible isotopologue for that species. Measurement of the abundance of each isotopologue can be conducted using multiple techniques such as mass spectrometry and/or laser-based spectroscopy.

At block 208 the temperature-dependent clumped isotope excess is compared with the previously determined stochastic distribution. Following measurement of the absolute abundance of co-existing isotopologues, it should be possible to integrate the modeled temperature dependence of isotopologues with the measured concentrations to (1) differentiate between hydrocarbons that originate directly from a source rock from those that have escaped from a subsurface accumulation, and (2) determine the current equilibrium storage temperature of the species in the reservoir prior to escape to the surface.

The differentiation between direct seepage from a source rock from the leakage of hydrocarbons from a subsurface accumulation requires consideration of the clumped isotopic signatures that may result from the two models of seepage. Hydrocarbons that have migrated directly from a source rock may either (i) retain a stochastic clumped isotope signature given insufficient time for a thermal contribution to the "clumping" of multiply substituted isotopologues, or (ii) display an inconsistent clumped isotope signature that arises as a result of the variability in the rate of isotope exchange of individual isotopologues. In contrast, hydrocarbons that derive from a subsurface accumulation will retain a clumped isotope signature that more consistently reflects the temperature at which they were stored in the subsurface. This non-kinetic control on the isotopic exchange reactions in isotopologues of hydrocarbons that originate from a subsurface accumulation arises as a result of the inherently long residence times of hydrocarbons in the subsurface. Aspects of the disclosed methodologies and techniques may thereby identify the presence of subsurface hydrocarbon accumulations. Once identified, it is possible to apply a suitable geothermal gradient to the equilibrium storage temperature to estimate the location (depth) within the subsurface that the associated hydrocarbon accumulation resides.

Another aspect of disclosed methodologies and techniques is characterizing the source rock from which the hydrocarbon originated. As represented by block 210, the results of previous portions of the method are integrated with known geochemical proxies that can be concomitantly used to determine the source facies by assessing the biomarker distribution of associated hydrocarbons and thermal maturity estimates through isotopic characterization of associated hydrocarbons. More specifically, from knowledge of the biomarker distribution of different organic matter sources and how this can be genetically linked to the hydrocarbons that are produced from such sources, it is possible to determine the source facies from which the accumulated hydrocarbon derived. In addition to this, from knowledge of how the isotopic signature of hydrocarbons from differently sourced organic matter evolves during maturation, it is possible to determine the thermal maturity of the source rock from which the hydrocarbons derive. The results of the method 200 may also be integrated with conventional exploration or prospect assessment technologies to confirm or de-risk the presence and/or location of a hydrocarbon accumulation and to assess potential migration pathways from the source rock to the seep. Such technologies may include reflection seismic, high resolution seismic imaging, acoustic, basin modeling, and/or probabilistic or statistical assessments. By integrating these technologies, various characteristics of the accumulation may be estimated, such as hydrocarbon volume, hydrocarbon type (e.g., oil vs. gas), and the like. Once a hydrocarbon accumulation has been identified and located, the hydrocarbons therein may be extracted or otherwise produced using known principles of hydrocarbon management.

An alternative to concomitantly determine the reservoir temperature, source facies, and thermal maturity may involve statistical regression analysis to converge on the temperature dependent equilibrium constant and uncommon isotopes, such as $^{13}C$ and D that may be unique to the relative concentrations reported for multiple co-existing isotopologues.

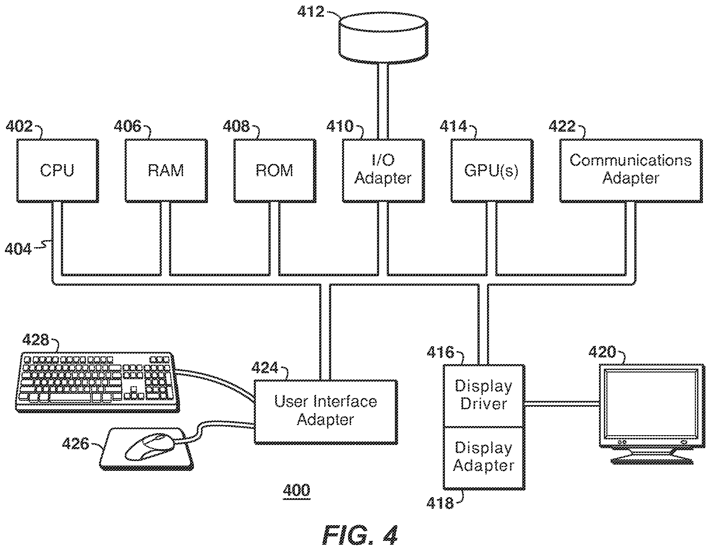

Figure 4 is a block diagram of a computer system 400 that may be used to perform some or all of the disclosed aspects and methodologies. A central processing unit (CPU) 402 is coupled to system bus 404. The CPU 402 may be any general-purpose CPU, although other types of architectures of CPU 402 (or other components of exemplary system 400) may be used as long as CPU 402 (and other components of system 400) supports the inventive operations as described herein. The CPU 402 may execute the various logical instructions according to various exemplary embodiments. For example, the CPU 402 may execute machine-level instructions for performing processing according to the operational flow described above. One or more Graphics Processing Units (GPU) 414 may be included and used as known in the art.

The computer system 400 may also include computer components such as a random access memory (RAM) 406, which may be SRAM, DRAM, SDRAM, or the like. The computer system 400 may also include read-only memory (ROM) 408, which may be PROM, EPROM, EEPROM, or the like. RAM 406 and ROM 408 hold user and system data and programs, as is known in the art. The computer system 400 may also include an input/output (I/O) adapter 410, a communications adapter 422, a user interface adapter 424, and a display adapter 418. The I/O adapter 410, the user interface adapter 424, and/or communications adapter 422 may, in certain embodiments, enable a user to interact with computer system 400 in order to input information.

The I/O adapter 410 preferably connects a storage device(s) 412, such as one or more of hard drive, compact disc (CD) drive, floppy disk drive, tape drive, etc. to computer system 400. The storage device(s) may be used when RAM 406 is insufficient for the memory requirements associated with storing data for operations of embodiments of the present techniques. The data storage of the computer system 400 may be used for storing information and/or other data used or generated as disclosed herein. The communications adapter 422 may couple the computer system 400 to a network (not shown), which may enable information to be input to and/or output from system 400 via the network (for example, the Internet or other wide-area network, a local-area network, a public or private switched telephony network, a wireless network, any combination of the foregoing). User interface adapter 424 couples user input devices, such as a keyboard 428, a pointing device 426, and the like, to computer system 400. The display adapter 418 is driven by the CPU 402 to control, through a display driver 416, the display on a display device 420. Information and/or representations pertaining to a portion of a supply chain design or a shipping simulation, such as displaying data corresponding to a physical or financial property of interest, may thereby be displayed, according to certain exemplary embodiments.

The architecture of system 400 may be varied as desired. For example, any suitable processor-based device may be used, including without limitation personal computers, laptop computers, computer workstations, and multi-processor servers. Moreover, embodiments may be implemented on application specific integrated circuits (ASICs) or very large scale integrated (VLSI) circuits. In fact, persons of ordinary skill in the art may use any number of suitable structures capable of executing logical operations according to the embodiments.

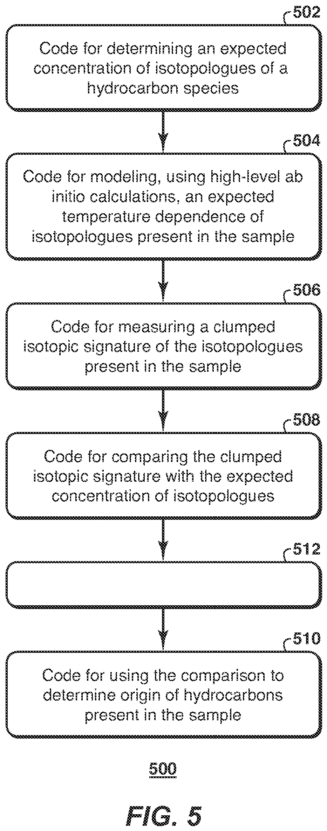

Figure 5 shows a representation of machine-readable logic or code 500 that may be used or executed with a computing system such as computing system 400. At block 502 code is provided for determining an expected concentration of isotopologues of a hydrocarbon species. At block 504 code is provided for modeling, using high-level ab initio calculations, an expected temperature dependence of isotopologues present in the sample. At block 506 code is provided for measuring a clumped isotopic signature of the isotopologues present in the sample. At block 508 code is provided for comparing the clumped isotopic signature with the expected concentration of isotopologues. At block 510 code is provided for using said comparison to determine whether hydrocarbons present in the sample originate directly from a source rock or whether the hydrocarbons present in the sample have escaped from a subsurface accumulation. When executed or applied with a computer system such as computer system 400, such code is configured to determine the presence and location of a subsurface hydrocarbon accumulation from a sample of naturally occurring substance. Code effectuating or executing other features of the disclosed aspects and methodologies may be provided as well. This additional code is represented in Figure 5 as block 512, and may be placed at any location within code 500 according to computer code programming techniques.

The disclosed methodologies and techniques may be susceptible to various modifications and alternative forms.

The scope of the invention is defined by the appended claims.

**Claims**

1. A method of determining a presence and location of a subsurface hydrocarbon accumulation from a sample of naturally occurring substance, the method comprising:

   determining an expected concentration of isotopologues of a hydrocarbon species;
   modeling, using high-level ab initio calculations, an expected temperature dependence of isotopologues present in the sample;
   measuring a clumped isotopic signature of the isotopologues present in the sample;
   integrating the modelled temperature dependence of isotopologues with the measured concentrations and comparing the clumped isotopic signature with the expected concentration of isotopologues;
   determining, using said comparison, and using any deviation between the expected and measured concentrations of isotopologues, whether hydrocarbons present in the sample originate directly from a source rock or whether the hydrocarbons present in the sample have escaped from a subsurface accumulation;
   determining, using said comparison, the current equilibrium storage temperature of the hydrocarbon species in the subsurface accumulation prior to escape to the surface; and

estimating a location of the subsurface accumulation by applying a geothermal gradient to the equilibrium storage temperature.

2. The method of claim 1, wherein determining an expected concentration of isotopologues includes determining a stochastic distribution of isotopologues of the hydrocarbon species for a given bulk isotopic signature for the species.

3. The method of claim 2, further comprising:

where the given bulk isotopic signature of the hydrocarbon species has been altered from secondary isotope exchange processes or from mixing, applying a correction scheme to arrive at an initial primary isotopic signature representative of what was produced from the source rock.

4. The method of claim 1, wherein the location comprises a depth.

5. The method of claim 1, further comprising determining a source facies from which the hydrocarbons in the subsurface accumulation derived.

6. The method of claim 5, wherein determining a source facies includes genetically linking biomarker distribution of organic matter sources to the hydrocarbons produced from the source facies.

7. The method of claim 1, further comprising determining a thermal maturity of the source rock from which hydrocarbons in the subsurface accumulation derive.

8. The method of claim 1, further comprising determining a location of the subsurface hydrocarbon accumulation using a geophysical imaging technique.

9. The method of claim 8, wherein the geophysical imaging technique is seismic reflection.

10. A computer system configured to determine a presence and location of a subsurface hydrocarbon accumulation from a sample of naturally occurring substance, the computer system comprising:

a processor; and

a tangible, machine-readable storage medium that stores machine-readable instructions for execution by the processor, the machine-readable instructions including

code for determining an expected concentration of isotopologues of a hydrocarbon species,

code for modeling, using high-level ab initio calculations, an expected temperature dependence of isotopologues present in the sample,

code for measuring a clumped isotopic signature of the isotopologues present in the sample,

code for integrating the modelled temperature dependence of isotopologues with the measured concentrations, and for comparing the clumped isotopic signature with the expected concentration of isotopologues,

code for determining, using said comparison, and using deviations between the expected and measured concentrations of isotopologues, whether hydrocarbons present in the sample originate directly from a source rock or whether the hydrocarbons present in the sample have escaped from a subsurface accumulation,

code for determining, using said comparison, the current equilibrium storage temperature of the hydrocarbon species in the subsurface accumulation prior to escape to the surface, and

code for estimating a location of the subsurface accumulation by applying a geothermal gradient to the equilibrium storage temperature.

11. The system of claim 10, wherein the code for determining an expected concentration of isotopologues includes code for determining a stochastic distribution of isotopologues of the hydrocarbon species for a given bulk isotopic signature for the species.

12. The system of claim 10, further comprising code for determining a source facies from which the hydrocarbons in the subsurface accumulation derived.

**Patentansprüche**

1. Verfahren zum Bestimmen eines Vorhandenseins und einer Lage einer unterirdischen Kohlenwasserstoffansammlung aus einer Probe von natürlich vorkommender Substanz, bei dem

eine erwartete Isotopologen-Konzentration einer Kohlenwasserstoffspezies bestimmt wird,
unter Verwendung von hochgenauen ("high-level") Ab-initio-Berechnungen eine erwartete Temperaturabhängigkeit von in der Probe vorhandenen Isotopologen modelliert wird,
von den in der Probe vorhandenen Isotopologen eine "verklumpte Isotopen"-Signatur ("clumped isotope signature") gemessen wird,
die modellierte Temperaturabhängigkeit von Isotopologen mit den gemessenen Konzentrationen integriert wird und
die "verklumpte Isotopen"-Signatur mit der erwarteten Isotopologen-Konzentration verglichen wird,
unter Verwendung des Vergleichs und unter Verwendung jeder Abweichung zwischen den erwarteten und gemessenen Isotopologen-Konzentrationen bestimmt wird, ob in der Probe vorhandene Kohlenwasserstoffe direkt aus einem Muttergestein stammen oder ob die in der Probe vorhandenen Kohlenwasserstoffe aus einer unterirdischen Ansammlung ausgetreten sind,
unter Verwendung des Vergleichs die derzeitige Gleichgewichtslagerungstemperatur der Kohlenwasserstoffspezies in der unterirdischen Ansammlung vor dem Austritt an die Oberfläche bestimmt wird und
eine Lage der unterirdischen Ansammlung durch die Anwendung eines geothermalen Gradienten an die Gleichgewichtslagerungstemperatur abgeschätzt wird.

2. Verfahren nach Anspruch 1, bei dem das Bestimmen einer erwarteten Isotopologen-Konzentration einschließt, dass eine stochastische Verteilung von Isotopologen der Kohlenwasserstoffspezies für eine für die Spezies vorgegebene Massen-Isotopensignatur ("bulk isotopic signature") bestimmt wird.

3. Verfahren nach Anspruch 2, bei dem ferner dort, wo die vorgegebene Massen-Isotopensignatur der Kohlenwasserstoffspezies durch sekundäre Isotopenaustauschprozesse oder durch Mischen geändert worden ist, ein Korrekturschema angewendet wird, um zu einer anfänglichen primären Isotopensignatur zu gelangen, die repräsentativ für das ist, was vom Muttergestein hergestellt wurde.

4. Verfahren nach Anspruch 1, bei dem die Lage eine Tiefe umfasst.

5. Verfahren nach Anspruch 1, bei dem ferner eine Fazies des Muttergesteins bestimmt wird, aus dem die Kohlenwasserstoffe in der unterirdischen Ansammlung stammen.

6. Verfahren nach Anspruch 5, bei dem das Bestimmen einer Fazies des Muttergesteins umfasst, dass eine Biomarkerverteilung von Quellen organischen Materials mit den Kohlenwasserstoffen, die von der Fazies des Muttergesteins hergestellt wurden, genetisch verknüpft werden.

7. Verfahren nach Anspruch 1, bei dem ferner eine thermische Reife des Muttergesteins bestimmt wird, aus dem die Kohlenwasserstoffe in der unterirdischen Ansammlung stammen.

8. Verfahren nach Anspruch 1, bei dem ferner unter Verwendung eines geophysikalischen Bildgebungsverfahrens eine Lage der unteririschen Kohlenwasserstoffansammlung bestimmt wird.

9. Verfahren nach Anspruch 8, bei dem das geophysikalische Bildgebungsverfahren Reflexionsseismik ist.

10. Computersystem, das ausgebildet ist, um ein Vorhandensein und eine Lage einer unterirdischen Kohlenwasserstoffansammlung aus einer Probe von natürlich vorkommender Substanz zu bestimmen, wobei das Computersystem einen Prozessor und
ein greifbares, maschinenlesbares Speichermedium umfasst, das maschinenlesbare Anweisungen zur Ausführung durch den Prozessor speichert, wobei die maschinenlesbaren Anweisungen

Code, um eine erwartete Isotopologen-Konzentration einer Kohlenwasserstoffspezies zu bestimmen, Code, um unter Verwendung von hochgenauen Ab-initio-Berechnungen eine erwartete Temperaturabhängigkeit von in der Probe vorhandenen Isotopologen zu modellieren,
Code, um eine "verklumpte Isotopen"-Signatur der in der Probe vorhandenen Isotopologen zu messen, Code, um die modellierte Temperaturabhängigkeit von Isotopologen mit den gemessenen Konzentrationen zu integrieren und um die "verklumpte Isotopen"-Signatur mit den erwarteten Isotopologen-Konzentrationen zu vergleichen,

Code, um unter Verwendung des Vergleichs und unter Verwendung von Abweichungen zwischen den erwarteten und gemessenen Konzentrationen von Isotopologen zu bestimmen, ob in der Probe vorhandene Kohlenwasserstoffe direkt aus einem Muttergestein stammen oder ob die in der Probe vorhandenen Kohlenwasserstoffe aus einer unterirdischen Ansammlung ausgetreten sind,

Code, um unter Verwendung des Vergleichs die derzeitige Gleichgewichtslagerungstemperatur der Kohlenwasserstoffspezies in der unterirdischen Ansammlung vor dem Austritt an die Oberfläche zu bestimmen, und

Code, um eine Lage der unterirdischen Ansammlung durch Anwendung eines geothermalen Gradienten an die Gleichgewichtslagerungstemperatur abzuschätzen,

einschließt.

11. System nach Anspruch 10, bei dem der Code zur Bestimmung einer erwarteten Isotopologen-Konzentration Code einschließt, um eine stochastische Verteilung von Isotopologen der Kohlenwasserstoffspezies für eine vorgegebene Massen-Isotopensignatur für die Spezies zu bestimmen.

12. System nach Anspruch 10, das zudem Code einschließt, um eine Fazies des Muttergesteins zu bestimmen, aus dem die Kohlenwasserstoffe in der unterirdischen Ansammlung stammen.

## Revendications

1. Procédé de détermination d'une présence et d'un emplacement d'une accumulation souterraine d'hydrocarbures à partir d'un échantillon de substance d'origine naturelle, le procédé comprenant :

la détermination d'une concentration prévue d'isotopologues d'une espèce d'hydrocarbures ;

la modélisation, au moyen de calculs ab initio de haut niveau, d'une dépendance en température prévue d'isotopologues présents dans l'échantillon ;

la mesure d'une signature isotopique agrégée des isotopologues présents dans l'échantillon ;

l'intégration de la dépendance en température modélisée d'isotopologues avec les concentrations mesurées et la comparaison de la signature isotopique agrégée avec la concentration prévue d'isotopologues ;

la détermination, au moyen de ladite comparaison, et au moyen de tout écart entre les concentrations prévue et mesurée d'isotopologues, que les hydrocarbures présents dans l'échantillon proviennent ou non directement d'une roche mère ou que les hydrocarbures présents dans l'échantillon se sont échappés ou non d'une accumulation souterraine ;

la détermination, au moyen de ladite comparaison, de la température de stockage à l'équilibre actuelle de l'espèce hydrocarbonée dans l'accumulation souterraine avant échappement à la surface ; et

l'estimation d'un emplacement de l'accumulation souterraine par application d'un gradient géothermique à la température de stockage à l'équilibre.

2. Procédé de la revendication 1, dans lequel la détermination d'une concentration prévue d'isotopologues comporte la détermination d'une distribution stochastique d'isotopologues de l'espèce hydrocarbonée pour une signature isotopique brute donnée pour l'espèce.

3. Procédé de la revendication 2, comprenant en outre :
lorsque la signature isotopique brute donnée de l'espèce hydrocarbonée a été modifiée par des processus d'échange isotopique secondaires ou par mélange, l'application d'un schéma de correction pour aboutir à une signature isotopique primaire initiale représentative de celle qui a été produite à partir de la roche mère.

4. Procédé de la revendication 1, dans lequel l'emplacement comprend une profondeur.

5. Procédé de la revendication 1, comprenant en outre la détermination d'un faciès source duquel sont provenus les hydrocarbures dans l'accumulation souterraine.

6. Procédé de la revendication 5, dans lequel la détermination d'un faciès source comporte l'association génétique d'une distribution de biomarqueurs de sources de matière organique avec les hydrocarbures produits à partir du faciès source.

7. Procédé de la revendication 1, comprenant en outre la détermination d'une maturité thermique de la roche mère de laquelle proviennent les hydrocarbures dans l'accumulation souterraine.

8. Procédé de la revendication 1, comprenant en outre la détermination d'un emplacement de l'accumulation souterraine d'hydrocarbures au moyen d'une technique d'imagerie géophysique.

9. Procédé de la revendication 8, dans lequel la technique d'imagerie géophysique est la réflexion sismique.

10. Système informatique configuré pour déterminer une présence et un emplacement d'une accumulation souterraine hydrocarbures à partir d'un échantillon de substance d'origine naturelle, le système informatique comprenant :

un processeur ; et
un support de stockage tangible lisible par machine qui stocke des instructions lisibles par machine pour exécution par le processeur, les instructions lisibles par machine comportant
un code pour déterminer une concentration prévue d'isotopologues d'une espèce d'hydrocarbures,
un code pour modéliser, en utilisant des calculs ab initio de haut niveau, une dépendance en température prévue d'isotopologues présents dans l'échantillon,
un code pour mesurer une signature isotopique agrégée des isotopologues présents dans l'échantillon,
un code pour intégrer la dépendance en température modélisée d'isotopologues avec les concentrations mesurées, et pour comparer la signature isotopique agrégée avec la concentration prévue d'isotopologues,
un code pour déterminer, en utilisant ladite comparaison, et en utilisant des écarts entre les concentrations prévue et mesurée d'isotopologues, si les hydrocarbures présents dans l'échantillon proviennent directement d'une roche mère ou si les hydrocarbures présents dans l'échantillon se sont échappés d'une accumulation souterraine,
un code pour déterminer, en utilisant ladite comparaison, la température de stockage à l'équilibre actuelle de l'espèce hydrocarbonée dans l'accumulation souterraine avant échappement à la surface, et
un code pour estimer un emplacement de l'accumulation souterraine en appliquant un gradient géothermique à la température de stockage à l'équilibre.

11. Système de la revendication 10, dans lequel le code pour déterminer une concentration prévue d'isotopologues comporte un code pour déterminer une distribution stochastique d'isotopologues de l'espèce hydrocarbonée pour une signature isotopique brute donnée pour l'espèce.

12. Système de la revendication 10, comprenant en outre un code pour déterminer un faciès source duquel sont provenus les hydrocarbures dans l'accumulation souterraine.

**FIG. 1**

EP 2 776 663 B1

202

Determine stochastic distribution of
isotopologues

204

Determine theoretical clumped isotopic
signature of each isotopologue

206

Measure clumped isotopic signature
of isotopologues

208

Compare temperature dependent
clumped isotope excess with
stochastic distribution

210

Integrate with geochemical
measurements to identify source
facies and maturity of source rock

200

FIG. 2

FIG. 3

FIG. 4

502

Code for determining an expected
concentration of isotopologues of a
hydrocarbon species

504

Code for modeling, using high-level ab
initio calculations, an expected
temperature dependence of
isotopologues present in the sample

506

Code for measuring a clumped
isotopic signature of the isotopologues
present in the sample

508

Code for comparing the clumped
isotopic signature with the expected
concentration of isotopologues

512

510

Code for using the comparison to
determine origin of hydrocarbons
present in the sample

500

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007008932 A2

**Non-patent literature cited in the description**

- **HOHL, D. ; MAILHIOT, C.** *DCO Energy, Environment and Climate Workshop,* 22 July 2010, 1-38
- **EILER, J.M.** *Clumped-isotope*
- *Earth and Planetary Science Letters,* 2007, 309-327

- **HUNTINGTON et al.** Use of clumped-isotope thermometry to constrain the crystallization temperature of diagenetic calcite. *Journal of Sedimentary Research.,* 2011
- **CHUNG et al.** Origin of gaseous hydrocarbons in subsurface environment: theoretical considerations of carbon isotope distribution. *Chemical Geology,* 1988, vol. 71, 97-104